(19)

Europäisches Patentamt
European Patent Office
Office européen des brevets

(11) **EP 4 607 462 A1**

(12) **EUROPEAN PATENT APPLICATION**

(43) Date of publication:
**27.08.2025 Bulletin 2025/35**

(21) Application number: **25158537.8**

(22) Date of filing: **18.02.2025**

(51) International Patent Classification (IPC):
***G06T 7/20*** *(2017.01)*

(52) Cooperative Patent Classification (CPC):
**G06T 7/20;** G06T 2207/10081; G06T 2207/20084;
G06T 2207/30004

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.02.2024 US 202418586533**

(71) Applicant: **Siemens Healthineers International AG
6312 Steinhausen (CH)**

(72) Inventors:
• **WALCZAK, Michal
5408 Ennetbaden (CH)**
• **ZHU, Liangjia
Menlo Park, CA 94025 (US)**

(74) Representative: **Mathisen & Macara LLP
Charta House
30-38 Church Street
Staines-upon-Thames TW18 4EP (GB)**

(54) **IMAGE DATA PROCESSING AND TARGET STRUCTURE TRACKING FOR RADIATION THERAPY**

(57) Example methods (100) and systems (300, 400) for image data processing for target structure tracking are described. In one example, a computer system may obtain treatment image data (140) associated with a target structure of a patient requiring radiation therapy. The treatment image data (140) may be acquired using an imaging system during a treatment phase (102) of the radiation therapy. The computer system may process (160) the treatment image data (140) using an artificial intelligence (AI) engine to generate material property data representing a particular material property associated with the target structure. The material property data may be generated to be matchable against a template that also represents the particular material property for tracking the target structure based on the particular material property during the treatment phase (102).

FIG. 1

**Description**

CROSS-REFERENCE TO RELATED APPLICATION

The present application is related in subject matter to U.S. Patent Application No. US18/586,532 (Attorney Docket No. 124-0068-US1), entitled "TEMPLATE GENERATION AND TARGET STRUCTURE TRACKING FOR RADIATION THERAPY".

BACKGROUND

Radiation therapy is a widely used cancer treatment modality that uses high-energy radiation to reduce or eliminate cancerous tumors. In practice, applied radiation does not inherently discriminate between a tumor and proximal healthy structures, such as organs, etc. Ideally, the objective is to deliver a lethal or curative radiation dose to the tumor, while maintaining an acceptable dose level in the proximal healthy structures. During treatment time, delivery of planned radiation dose may be hindered by the presence of patient motion. In this case, motion management may be performed to maintain substantially high precision in radiation therapy by tracking the position of a moving target structure and acting upon any deviation from a planned position. Conventionally, one approach for target structure tracking involves tracking fiducial markers/transponders that have been implanted into patients. However, the implantation of such markers/transponders may carry additional risks. In some cases, after implantation, some markers might migrate and become unreliable.

SUMMARY

In accordance with a first aspect of the invention, there is provided a method for a computer system to perform image data processing for tracking of an anatomical structure of a subject, as defined by claim 1.
In accordance with a second aspect of the invention, there is provided a computer system as defined by claim 11.
Optional features are defined by the dependent claims.
According to examples of the present disclosure, target structure tracking may be implemented using a template-based, markerless approach. To improve tracking accuracy, target structure tracking may be implemented based on a particular material property associated with target structure(s). In practice, examples of the present disclosure may be implemented to improve positional verification, dose accuracy and conformity and sparing of healthy tissue during a treatment phase of radiation therapy. As will be described further below, examples of the present disclosure should be contrasted against conventional approaches that rely on implanted fiducial markers and/or comparison of different material properties during target structure tracking.
As used herein, the term "material property" may refer generally to a quantifiable characteristic based on which a target structure is trackable according to examples of the present disclosure, such as material density/thickness, effective atomic number, etc. In practice, the material property may be a characteristic that influences a material's interaction with ionizing radiation (e.g., X-rays, proton therapy) during radiation therapy. The term "target structure tracking" may refer generally to estimating position data associated with a target structure, such as to facilitate position monitoring and/or verification, target localization or the like during a treatment phase of radiation therapy. The term "target structure" may refer generally to any suitable structure that requires tracking, such as tumor, organ-at-risk (OAR), healthy tissue, bony structure (e.g., vertebra), etc.

*Method*

In accordance with the first aspect of the invention, there is disclosed a method for a computer system to perform image data processing for tracking of an anatomical structure of a subject, wherein the method comprises:

obtaining image data associated with the anatomical structure of the subject, wherein the image data is acquired using an imaging system;
processing the image data using an artificial intelligence (AI) engine to generate material property data representing a particular material property associated with the anatomical structure, wherein the material property data is matchable against a template that also represents the particular material property for tracking the anatomical structure based on the particular material property.

The method may further comprise, based on the material property data and the template, performing template matching to track the anatomical structure based on the particular material property.

*System*

In accordance with the second aspect of the invention, there is provided a computer system, comprising:

a processor; and
a non-transitory computer-readable medium having stored thereon instructions that, when executed by the processor, cause the processor to perform the following:

obtain image data associated with a an anatomical structure of a subject, wherein the image data is acquired using an imaging system;
process the image data using an artificial intelligence (AI) engine to generate material property data representing a particular material property associated with the anatomical structure, wherein the material property data is matchable against a template that also represents the particular material property for tracking the anatomical structure based on the particular material property during the treatment phase.

The system may be further configured to, based on the material property data and the template, performing template matching to track the anatomical structure based on the particular material property.

(a) Template generation and target structure tracking

Examples of the present disclosure provide method(s) and system(s) for template generation for radiation therapy. In one example, a first computer system may obtain (a) planning image data associated with a target structure of a patient requiring radiation therapy and/or (b) transformed image data. The planning image data may be acquired prior to a treatment phase of the radiation therapy. The transformed image data may be generated based on the planning image data. Based on the planning image data and/or the transformed image data, the first computer system may generate first material property data that represents a particular material property associated with the target structure. Based on the first material property data, the first computer system may generate a template that represents the particular material property. The template may be generated to be matchable against second material property data that also represents the particular material property for tracking the target structure during the treatment phase.

Examples of the present disclosure provide method(s) and system(s) for target structure tracking based on template(s) representing a particular material property. In one example, a second computer system may obtain material property data that represents a particular material property associated with a target structure of a patient requiring radiation therapy. The material property data may be generated based on treatment image data acquired during a treatment phase of the radiation therapy. The second computer system may obtain a template that also represents the particular material property associated with the target structure. The template may be generated based on (a) planning image data that is acquired prior to the treatment phase or (b) transformed image data that is generated based on the planning image data. Based on the material property data and the template, the second computer system may perform template matching during the treatment phase to track the target structure based on the particular material property.

(b) Image data processing and target structure tracking

Examples of the present disclosure provide method(s) and system(s) for image data processing for radiation therapy. In one example, a second computer system may obtain treatment image data associated with a target structure of a patient requiring radiation therapy, wherein the treatment image data is acquired using an imaging system during a treatment phase of the radiation therapy. The second computer system may process the treatment image data using an artificial intelligence (AI) engine to generate material property data representing a particular material property associated with the target structure. This way, the material property data may be generated to be matchable against a template that also represents the particular material property for tracking the target structure based on the particular material property during the treatment phase.

Examples of the present disclosure provide method(s) and system(s) for target structure tracking using the output of image data processing. In one example, a second computer system may obtain material property data that represents a particular material property associated with a target structure of a patient requiring radiation therapy. The material property data may be generated using an AI engine based on treatment image data acquired during a treatment phase of the radiation therapy. The second computer system may obtain a template that also represents the particular material property associated with the target structure. Here, the template may be generated based on (a) planning image data that is acquired prior to the treatment phase or (b) transformed image data that is generated based on the planning image data. Based on the material property data and the template, the second computer system may perform template matching during the treatment phase to track the target structure based on the particular material property.

BRIEF DESCRIPTION OF DRAWINGS

FIG. 1 is a flow diagram illustrating an example overview process for target structure tracking for radiation therapy using a template-based, markerless approach;

FIG. 2 is a flow diagram illustrating an example process for target structure tracking based on a particular material property;

FIG. 3 is a schematic diagram illustrating a first example radiation therapy system for planning image data acquisition and processing during a planning phase of radiation therapy;

FIG. 4 is a schematic diagram illustrating a second example radiation therapy system for treatment image data acquisition and processing during a treatment phase of radiation therapy;

FIG. 5 is a flow diagram illustrating a detailed example process for target structure tracking based on material property in the form of material density or material thickness;

FIG. 6 is a chart illustrating example fitted models for generating first material property data in the form of material density volume data in the example in FIG. 5;

FIG. 7 is a flow diagram illustrating a detailed example process for image data processing using an artificial intelligence (AI) engine to generate second material property data in the example in FIG. 5;

FIG. 8 is a flow diagram illustrating a detailed example process target structure tracking based on material property in the form of effective atomic number; and

FIG. 9 is a flow diagram illustrating a detailed example process for image data processing using an AI engine to generate second material property data in the example in FIG. 8.

DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented here. It will be readily understood that the aspects of the present disclosure, as generally described herein, and illustrated in the drawings, can be arranged, substituted, combined, and designed in a wide variety of different configurations, all of which are explicitly contemplated herein. Although the terms "first" and "second" are used to describe various elements, these elements should not be limited by these terms. These terms are used to distinguish one element from another. For example, a first element may be referred to as a second element, and vice versa. Independent of the grammatical term usage, individuals with male, female or other gender identities are included within the term.

Overview

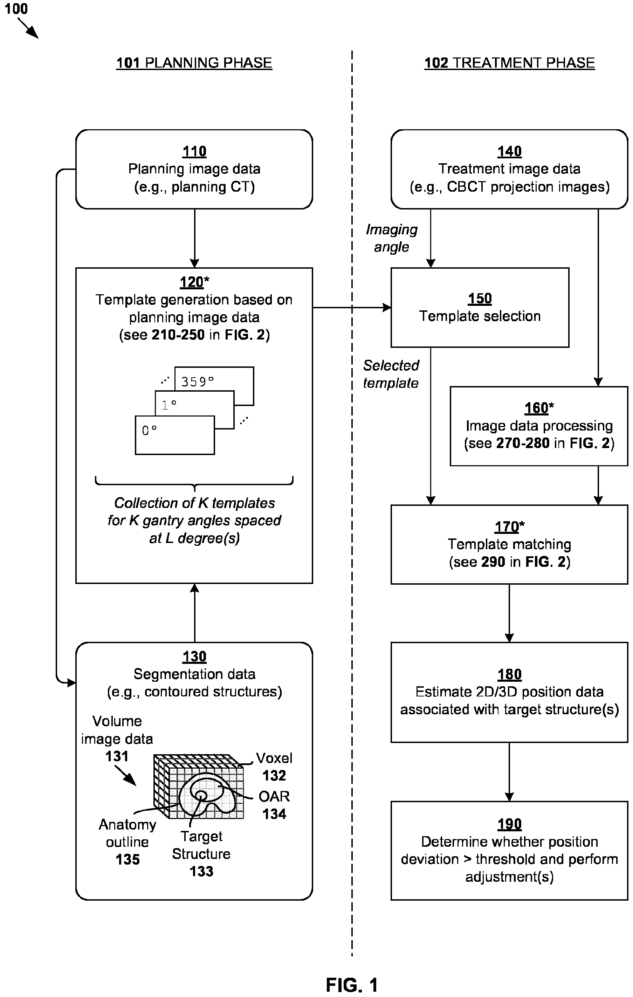

FIG. 1 is a flow diagram illustrating example overview process 100 for target structure tracking for radiation therapy using a template-based, markerless approach. Example process 100 may include one or more operations, functions, or actions illustrated by one or more blocks. The various blocks may be combined into fewer blocks, divided into additional blocks, and/or eliminated based upon the desired implementation. In the example in FIG. 1, radiation therapy may include (a) a pre-treatment planning phase (see 101) to generate a treatment plan for a patient requiring radiation therapy and (b) a treatment phase (see 102) to deliver treatment according to the treatment plan. The goal of the treatment plan is to deliver high radiation dose to a target structure (e.g., lung tumor) and lower radiation dose to proximal organs-at-risk (OARs) and healthy tissues (e.g., central airway).

During treatment phase 102, one important factor for effective treatment delivery is the location of the target structure within a planning target volume (PTV) to which high radiation dose is delivered. In practice, the patient or the tumor may move outside of the PTV due to intra-fraction motion (e.g., respiratory motion, patient's movement, etc.). One way of managing motion is tracking the target structure using a template-based, markerless approach that does not rely on invasive metal markers/transponders implanted into the patient. In the example in FIG. 1, markerless target structure tracking may involve (a) template generation during planning phase 101 and (b) template matching during treatment phase 102. This way, any deviation from a planned position may be detected during treatment delivery.

In more detail, at 110-130 in FIG. 1, template generation may involve generating a set of $K$ templates based on planning image data 110, such as planning computed tomography (CT) data that is acquired using a first imaging modality (i.e., CT) during planning phase 101, etc. For example, in order to be able to track from all gantry angles, a set of $K$ template images may be generated for $K = 360$ gantry angles that are spaced at $L = 1$ degree. Any additional data may be used for template generation, such as segmentation data 130 relating to contoured surfaces (e.g., drawn by a physician or determined using software/AI engine(s) for segmentation), etc. For example, segmentation may be performed to generate volume image data 131 identifying the contour, shape, size, and location of patient's anatomy 135, target structure 133 (e.g., tumor), OAR 134, or any other structure of interest (e.g., soft tissue, bone). Volume image data 131 (also known as a digital or treatment volume) may be divided into multiple smaller volume-pixels (voxels) 132, each representing a 3D element within the treatment volume. Note that volume image data 131 may include multiple target structures and irregularly shaped voxels. Depending on the desired implementation, data acquired during treatment phase 102 may also be used for template generation. Using adaptive therapy, for example, planning data from planning CT may be propagated to cone-based computed tomography (CBCT) of the day of treatment. This way, template(s) may be generated based on the CBCT volume, each template being a container to encode planning data from planning images or their variants via adaptation.

At 140 in FIG. 1, treatment image data associated with target structure 133 of the patient may be continuously acquired during irradiation, such as using a treatment delivery machine that includes an imaging system to facilitate target structure tracking. The imaging system may be capable of acquiring treatment image data 140 in the form of kilovoltage (kV) projection images during treatment delivery using any suitable treatment machine(s). For example, kV projection images may be acquired during volumetric modulated arc therapy (VMAT), where a gantry of the treatment delivery machine continuously rotates around the patient. In another example, kV projection images may be acquired using proton treatment machine, which deliver treatment using protons instead of X-ray radiation. In practice, treatment image data 140 may include two dimensional (2D) projection image data, such as single energy (SE) or dual energy (DE) CBCT projection image data, etc.

At 150 in FIG. 1, template selection may include selecting a template that is associated with the gantry angle closest to an imaging angle of treatment image data 140. At 160, any suitable image data processing to improve the quality of treatment image data 140 may be performed prior to subsequent template matching. At 170, during template matching, any suitable approach may be implemented to identify the best match between the selected template and treatment image data 140, such as calculating the normalized cross-correlation of all possible template locations within a specified search region on treatment image data 140 as a measure of similarity. The resulting match of template matching 170 may include the current 2D position data of target structure 133.

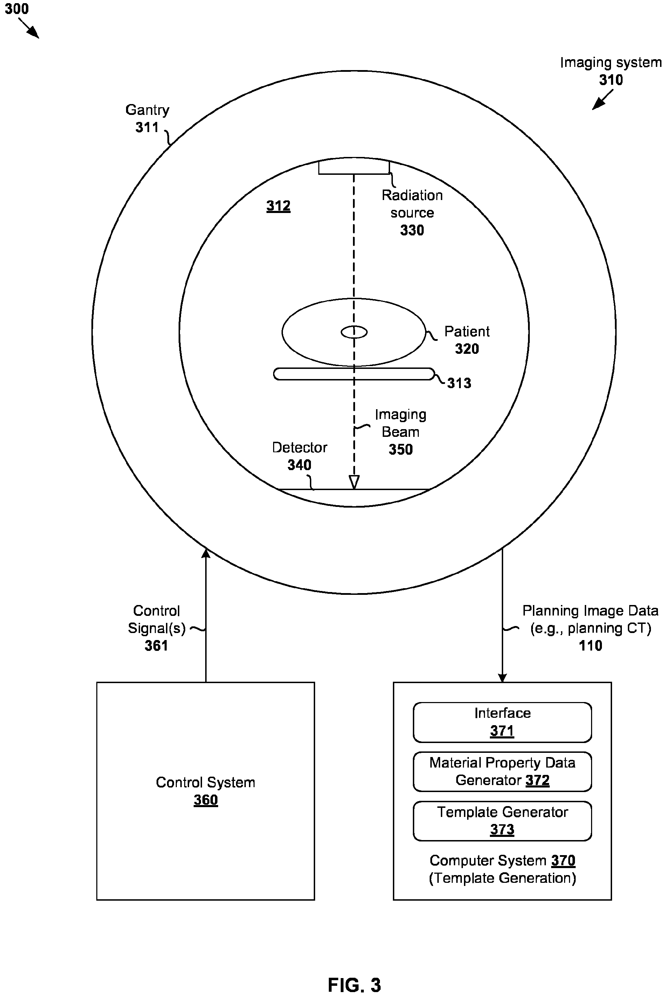

At 180 in FIG. 1, 3D position data associated with target structure 133 may be estimated using any suitable approach. In the case of monoscopic acquisition (e.g. using a single kV imager), triangulation may be performed based on the current 2D position data as well as the 2D position data associated with previous gantry angles. In the case of stereoscopic acquisition using two kV imagers, the instant 3D position may be estimated without using 2D position data from previous gantry angles. This way, the 3D position of target structure 133 may be monitored and verified as treatment delivery is performed during treatment phase 102. At 190, any adjustment may be performed in response to detecting an excessive positional displacement (e.g., threshold exceeded), such as to interrupt the treatment and/or recommend a patient repositioning, etc.

Target structure tracking based on material property

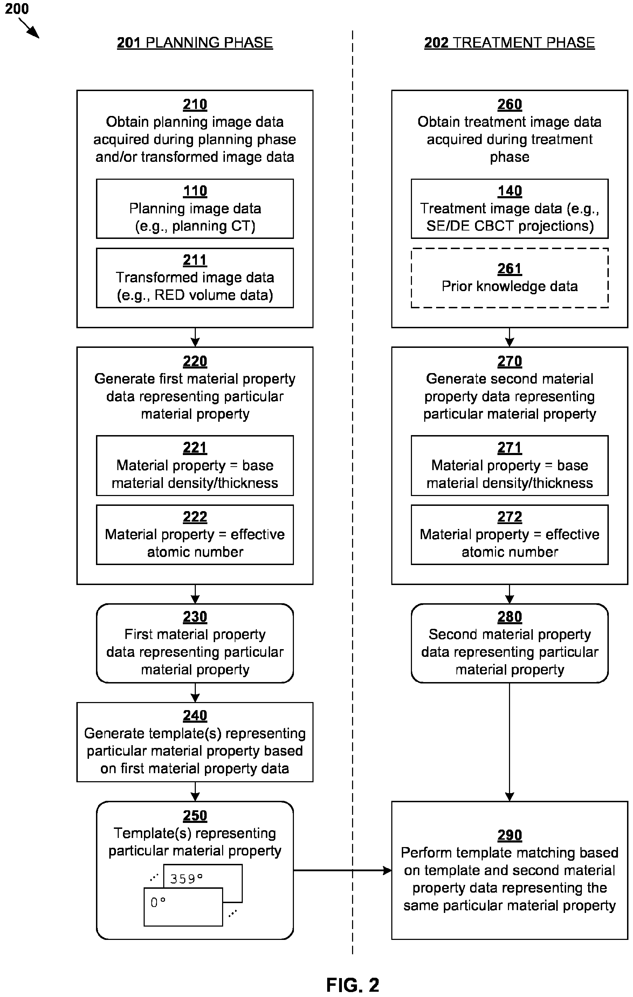

Blocks 120, 160-170 in FIG. 1 will be explained further using FIG. 2. According to examples of the present disclosure, target structure tracking may be performed based on template(s) and material property data that both represent the same material property associated with target structure 133. In particular, during planning phase 101, template(s) representing a particular material property associated with target structure 133 may be generated. During treatment phase 102, treatment image data 140 may be processed to generate material property data that also represents the particular material property. This way, tracking accuracy and dose conformity may be improved by performing template matching based on template(s) and material property data that represent the same physical quantity.

Examples of the present disclosure should be contrasted against conventional approaches that involve template matching based on different physical quantities, which may lead to less accurate results. For example, one conventional approach may involve generating templates by forward projecting a masked target region from a planning CT volume (i.e., planning image data 110 acquired using a first imaging modality). These templates represent a different physical quantity as measured in 2D CBCT projections (i.e., treatment image data 140 acquired using a second imaging modality). Another conventional approach may involve using dual energy acquisitions to create enhanced soft tissue or bone 2D projection images. However, the enhanced tissue images represent a different quantity compared to templates generated from the CT volume.

FIG. 2 is a flow diagram illustrating example process 200 for target structure tracking based on a particular material property. Example process 200 may include one or more operations, functions, or actions illustrated by one or more blocks. The various blocks may be combined into fewer blocks, divided into additional blocks, and/or eliminated based upon the desired implementation. According to example process 200, template generation (see blocks 210-250) may be performed during planning phase 201. Image data processing (see blocks 260-280) and template matching (see block 290) may be performed during treatment phase 202.

(a) Template generation

In more detail, at 210 in FIG. 2, planning image data 110 associated with target structure 133 and/or transformed image data 211 may be obtained. As used herein, the term "obtain" or "obtaining" may refer generally to receiving or retrieving data from any suitable source, such as an imaging system, another module/component on the same computer system, another computer system or a datastore capable of storing the data. Planning image data 110 may be acquired during planning phase 201 using any suitable radiation therapy system, an example of which will be described using FIG. 3. Transformed image data 211 may be generated based on planning image data 110, such as physical property data in the form of any combination of one or more of the following: relative electron density (RED) volume data, effective atomic number data, contrast agent map data (e.g., iodine map), etc. As used herein, the term "transformed image data" may refer generally to image data that is generated based on the planning image data by performing any suitable transformation, such as conversion from source values in the planning image data to target values in the transformed image data, etc. Although various examples will be described using RED volume data below, it should be understood that transformed image data 211 may include any additional and/or alternative physical property data.

At 220-230 in FIG. 2, based on planning image data 110 and/or transformed image data 211, first material property data 230 representing a particular material property may be generated. Any suitable "material property" based on which target structure 133 is trackable may be used, such as base material density or thickness (see 221), effective atomic number (see 222), etc. At 240-250, based on first material property data 230, template(s) 250 representing the particular material property may be generated for tracking the target structure. Here, the template(s) may be generated to be matchable against second material property data (see 270-280) that also represents the particular material property during subsequent template matching (see 290).

(b) Image data processing using AI engine(s)

At 260 in FIG. 2, treatment image data 140 associated with target structure 133 of a patient requiring radiation therapy may be obtained. At 270, treatment image data 140 may be processed using an AI engine to generate second material property data 280 representing a particular material property associated with target structure 133. The material property data may be generated to be matchable against a template that also represents the particular material property for tracking the target structure based on the particular material property during treatment phase 202. Any suitable "material property" based on which target structure 133 is trackable may be used, such as base material density or thickness (see 271), effective atomic number (see 272), etc.

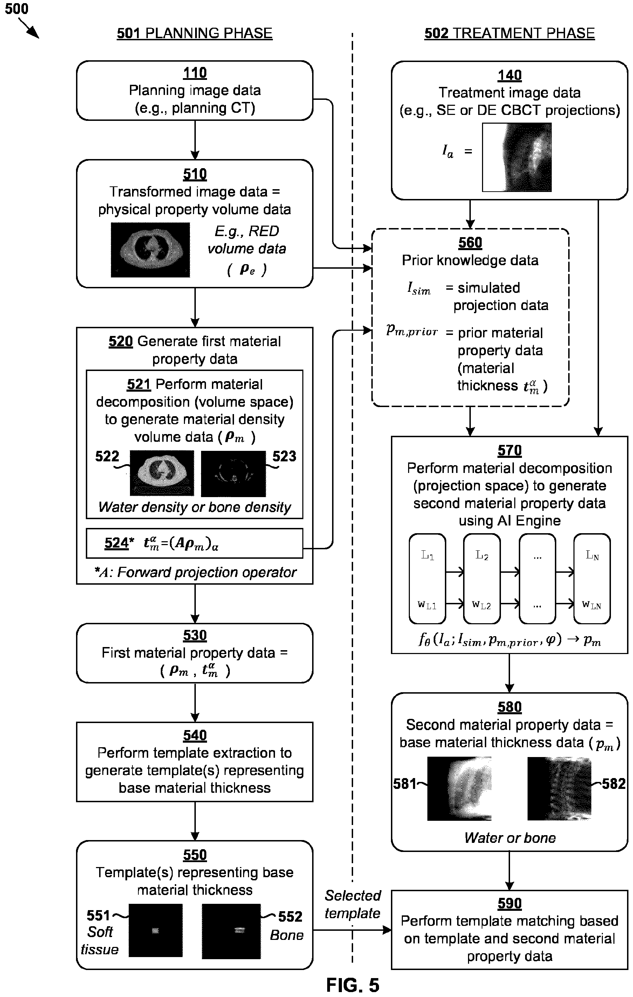

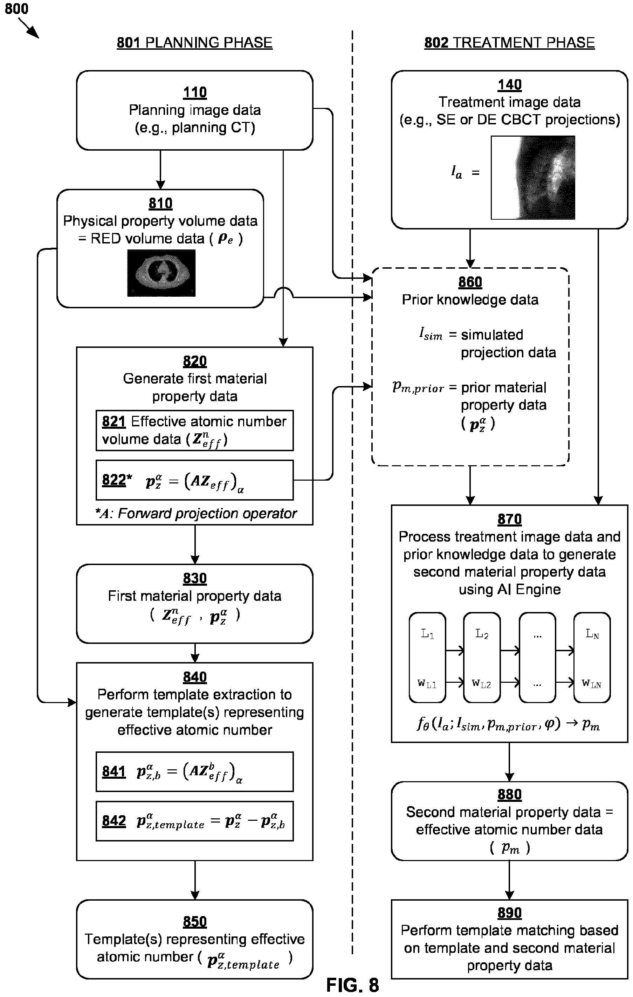

Depending on the desired implementation, the AI engine may be trained to map input = treatment image data 140 and prior knowledge data 261 to output = second material property data 280. As will be described further below, prior knowledge data 261 (see also 560 in FIG. 5 and 860 in FIG. 8) may include (a) simulated projection image data that is generated by performing polychromatic simulation based on transformed image data 211 and (b) projected material property data (e.g., first material property data 230) that is generated during planning phase 201. Prior knowledge data 261 represents *a priori* source of information to improve the result of the AI engine. In practice, transformed image data 211 may be generated based on planning image data 110. Depending on the desired implementation, transformed image data 211 may include any combination of one or more of the following physical property data: RED volume data, effective atomic number data, contrast agent map data (e.g., iodine map), etc.

(c) Target structure tracking

At 290 in FIG. 2, template matching may be performed to track target structure 133 based on template(s) 250 and second material property data 280 that both represent the same particular material property. Depending on the desired implementation, prior knowledge data (see 261) that is generated based on treatment image data 140 may be additionally obtained and processed using the AI engine to generate second material property data 280.

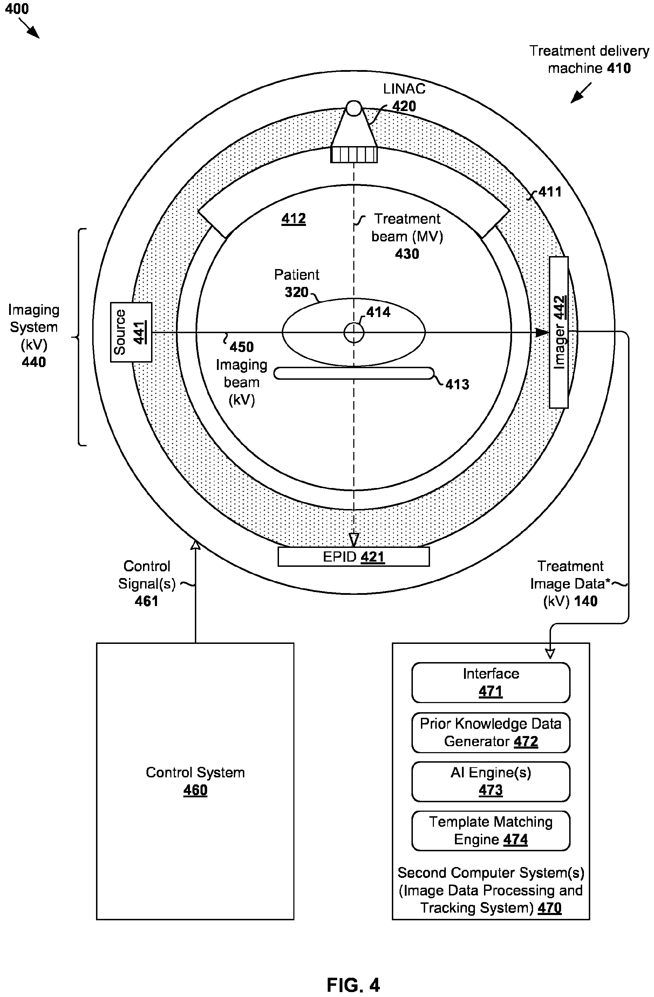

In the following, detailed examples relating to template generation, image data processing and target structure tracking based on template(s) 250 will be described below using FIGs. 3-9. In particular, an example first computer system for template generation will be described using FIG. 3 and example second computer system for image data processing and target structure tracking using FIG. 4. Examples relating to material property = material density/thickness will be described using FIGs. 5-7. Examples relating to material property = effective atomic number will be described using FIGs. 8-9.

Example imaging and computer systems

(a) Planning image data acquisition and processing

FIG. 3 is a schematic diagram illustrating first example radiation therapy system 300 for planning image data acquisition and processing during a planning phase of radiation therapy. It should be understood that, depending on the desired implementation, first example system 300 may include additional and/or alternative components than that shown in Fig. 3. Here, first example system 300 may include imaging system 310 to acquire planning image data 110, control system 360 to control operations of imaging system 310 and first computer system 370 to process planning image data 110 according to examples of the present disclosure.

In the example in FIG. 3, imaging system 310 may include gantry 311 having opening 312 and patient support 313 for supporting patient 320 requiring radiation therapy. Imaging system 310 may implement any suitable imaging modality for image data acquisition, such CT, positron emission tomography (PET), single photon emission computed tomography (SPECT), magnetic resonance imaging (MRI), magnetic resonance tomography (MRT), any combination thereof, etc. For example, when CT is used, planning image data 110 (e.g., planning CT scan) may include a series of 2D projection images or slices (e.g., CT slices), each representing a cross-sectional view of the patient's anatomy. For treatment planning, planning image data may include 3D volumetric CT data that is used (sometimes in combination with four dimensional (4D) CT) to estimate the motion range of target structure(s). In practice, spectral CT data (e.g., dual energy CT (DECT) and photon counting CT) may be acquired to provide access to various quantities at the planning stage.

In the example in FIG. 3, gantry 311 has a ring-based configuration. In an alternative example, gantry may have a C-arm configuration. Imaging system 310 may further include radiation source 330 (e.g., X-ray source) to project imaging beams 350 towards detector 340 having pixel detectors disposed opposite of radiation source 330. Control system 360 may be electrically coupled to gantry 311 to control operation(s) of gantry 311 using control signal(s) 361. Radiation source 330 may be configured to generate any suitable beam, such as fan beam, etc. During an imaging procedure, gantry 311 may be rotated about opening 312 while radiation source 330 generates and directs X-ray beam(s) 350 along a projection line towards patient 320 and detector 340. Detector 340 may measure the X-ray absorption and produce a voltage proportional to the intensity of incident X-rays. The voltage may be read and digitized to generate planning image data 110. In practice, planning image data 110 may include image data acquired at different gantry angles.

According to examples of the present disclosure, first computer system 370 may be coupled with imaging system 310 to obtain and process planning image data 110 for template generation according to blocks 210-250 in FIG. 2. In the example in FIG. 3, first computer system 370 may include interface 371 to interact with imaging system 310 to obtain planning image data 110; material property data generator 372 to generate first material property data 230; and template generator 373 to generate template(s) based on first material property data 230. First computer system 370 may include any alternative and/or additional components not shown in FIG. 3. In practice, first computer system 370 may be implemented using a physical machine (bare metal machine) and/or virtual machine that is deployed in a cloud-based environment (i.e., not located in the same physical location as imaging system 310).

(b) Treatment image data acquisition and processing

FIG. 4 is a schematic diagram illustrating second example radiation therapy system 400 for treatment image data acquisition and processing during a treatment phase of radiation therapy. It should be understood that, depending on the desired implementation, example system 400 may include additional and/or alternative components than that shown in Fig. 4. Here, example system 400 may include (a) treatment delivery machine 410 to deliver treatment to patient 320; (b) control system 460 to control operations of machine 410; and (c) second computer system 470 to process treatment image data 140 according to examples of the present disclosure.

Treatment delivery machine 410 may include gantry 411 that is rotatable about opening 412 and patient support 413 (e.g., treatment couch) for supporting patient 320. Note that gantry 411 may have a ring-based configuration (shown in FIG. 4) or C-arm configuration (not shown). Treatment delivery machine 410 may include a radiation source in the form of linear accelerator (LINAC) 420 as well as an imager/detector in the form of mega-electron volts (MV) electronic portal imaging device (EPID) 421. LINAC 420 may be configured to generate and direct treatment beam 430 towards isocenter 414 through a PTV associated with patient 320 as gantry 411 is rotated through a treatment arc during VMAT. In practice, treatment beam 430 may be within a high-energy range, such as 1 MV or greater. In practice, radiation therapy may be delivered as a fractionated treatment, where the total radiation dose to be delivered to a tumor is divided into smaller "fractions." This is to allow healthy cells to recover in between fractions from the damage caused by radiation, while tumor cells that are less efficient at recovering may accumulate damage.

Treatment delivery machine 410 may further include imaging system 440 to facilitate kV imaging during application of MV treatment beam 430. Any suitable image modality or modalities may be used, such as SE or DE CBCT, etc. Imaging system 440 may include at least one kV imaging source 441 and at least one kV imager 442. Compared to LINAC 420, kV imaging source 441 may be capable of producing imaging or diagnostic energy in the range of kV. During treatment delivery, control system 460 may configure kV imaging source 441 to emit and direct kV imaging beam 450 towards imager 442, thereby generating treatment image data 140 in the form of kV projection image data to facilitate markerless target structure tracking. Although described with reference to MV LINAC 420 and MV treatment beam 430, it should be understood that any additional or alternative treatment delivery technique(s) may be used. For example, a proton treatment machine that includes a kV imaging system may be used instead.

According to examples of the present disclosure, second computer system 470 may be coupled with treatment delivery machine 410 to obtain and process treatment image data 140 according to blocks 260-290 in FIG. 2. In the example in FIG. 4, second computer system 470 may include interface 471 to interact with imaging system 440 to obtain treatment image data 140; prior knowledge data generator 472 to generate prior knowledge data 261; AI engine(s) 473 to generate second material property data 280; and template matching engine 474 to perform template matching based on second material property data 280. In practice, AI engine(s) 473 and template matching engine 474 may be implemented by the same computer system (as shown in FIG. 4), or different computer systems. The term "computer system" may refer generally to one or more physical machines (bare metal machines) and/or virtual machines deployed in a cloud-based environment (i.e., not located in the same physical location as treatment delivery machine 410). Some examples will be described using FIGs. 5-9.

Template generation (base material density/thickness)

A first example material property is base material density/thickness. In the following, an example template generation will be described using FIG. 5, which is a flow diagram illustrating detailed example process 500 for target structure tracking based on material property in the form of material density or material thickness. Example process 500 may include one or more operations, functions, or actions illustrated by one or more blocks. The various blocks may be combined into fewer blocks, divided into additional blocks, and/or eliminated based upon the desired implementation.

In the example in FIG. 5, during planning phase 501, first computer system 370 may be configured to perform template generation based on first material property data according to blocks 510-550 (i.e., related to 210-250 in FIG. 2). During treatment phase 502, second computer system 470 may be configured to perform image data processing and template matching according to blocks 560-590 (i.e., related to 260-290 in FIG. 2). First computer system 370 in FIG. 3 may be deployed to implement blocks 510-550, and second computer system 470 in FIG. 4 to implement 560-590. Using the example in FIG. 5, target structure tracking may be performed in an improved manner based on material property in the form of base material density/thickness.

(a) Transformed image data

At 510 in FIG. 5, based on planning image data 110 (e.g., planning CT data) acquired using imaging system 310 shown in FIG. 3, computer system 370 may generate transformed image data, such as physical property volume data in the form of RED volume data, etc. In practice, RED volume data 510 is usually generated to facilitate dose calculation during planning phase 501, and hence is also available for template generation. In practice, the term "electron density" may refer generally to the number of electrons per unit volume in a material. RED volume data 510 may be a 3D representation of the electron density distribution within a patient's anatomy, usually expressed as a normalized value relative to water. The electron density of a given material influences how radiation interacts with the material, thereby impacting the amount of energy deposited and the types of interactions that may occur.

The physical property volume data may be obtained from spectral CT (e.g., DECT scan and photon counting CT), DirectDensity® CT reconstruction (registered trademark of Siemens Healthcare GmbH), polychromatic CBCT reconstruction, etc. For example, the generation of RED volume data 510 may involve transformation or conversion from CT Hounsfield Unit (HU) values to RED values, such as using suitable calibration curve(s), etc. Unlike HU, RED is a well-defined material property and does not depend on the kilovoltage peak (kVp) setting in CT acquisition. Depending on the desired implementation, HU-RED calibration may not be necessary, such as when DirectDensity is used, etc. Further, RED volume data 510 may be obtained in polychromatic CBCT reconstruction to reflect the current anatomy at the day of the treatment in adaptive radiation therapy (especially for soft tissue tracking).

(b) First material property data

At 520 in FIG. 5, based on RED volume data 510, computer system 370 may generate first material property data that includes (a) material density volume data 521 (denoted as $\rho_m$) and (b) projected material thickness data 524 (denoted as $t_m^{\alpha}$). For example, block 521 may involve performing material decomposition in the volume space (i.e., 3D) to map RED volume data 510 into base material density volume data 521, which includes one or more of the following: water density volume data (see 522), bone density volume data (see 523), etc. The particular base material(s) may be selected based on the target anatomy type requiring radiation therapy, such as bone material for vertebrae tracking, water material for lung tumor tracking, etc. Projected material thickness data 524 (2D) may be generated by performing a forward projection based on material density volume data 521 (3D) as follows: $t_m^{\alpha} = (A\rho_m)_{\alpha}$, where $A$ denotes a forward projector and $\alpha$ denotes a particular gantry angle.

Depending on the desired implementation, the basis for the volume material decomposition may include a physical model that reproduces X-ray attenuation properties of a given material (e.g., human tissue such as muscle and adipose, or common metal implant) as a combination of selected base materials, such as water, bone, titanium alloy, etc. In one example, input RED volume data 510 may be transformed into base material density volume data 521 using a polychromatic attenuation model. Given attenuation curves for the base materials (e.g., water and cortical bone), the model may optimize scaling factor(s) of the base material mass densities, such that the resulting linear combination of the base material attenuation curves is close to the attenuation curve of a selected material (e.g., muscle tissue).

For example, to account for spectral properties associated with imaging system 310 in FIG. 3, the difference between attenuation curves in the optimization process may be weighted by system sensitivity data reflecting the source spectrum and the detector response. Next, the base material scaling factors may be calculated for different human tissues and typical metal implant materials. In practice, patient 320 may have metal implants (e.g., for hip replacement) in which case the implant material may be appropriately modelled in the polychromatic simulation. A piecewise linear model may then be fitted such as to map RED values of tissue and implant materials to base material densities. Thus, by construction, the model may be used to account for spectral properties of both imaged tissues as well as specific characteristics of imaging system 310 used for acquiring the 2D projections (i.e., planning image data 110).

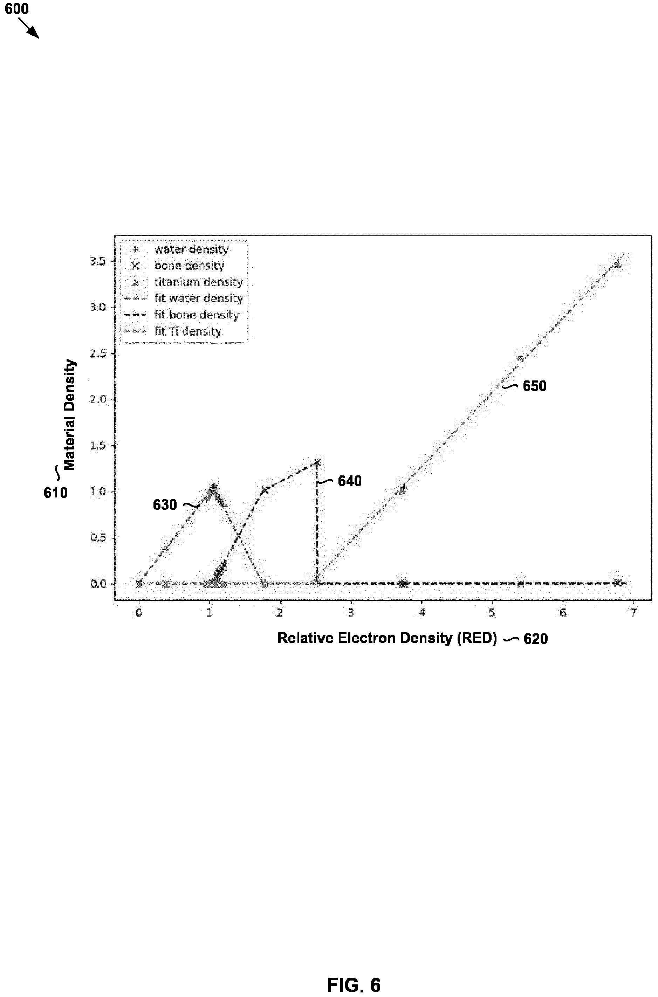

Some examples are shown in FIG. 6, which is a chart illustrating example fitted models 600 for generating first material property data in the form of base material volume data 530. In this example, the x-axis (see 610) represents input = RED values and the y-axis (see 620) represents output = base material density values, such as water density, bone density and titanium density. Individual marker points represent different tissue types or metal implant materials ordered according to their RED values, such as "+" representing water density, "x" representing bone density and "Δ" representing metal (i.e., titanium) density. Dashed lines (see 630-650) represent different fitted models that may be used by first computer system 370 to perform material decomposition. First fitted model 630 is for mapping RED values to water density values. Second fitted model 640 is for mapping to bone density values. Third fitted model 650 is for mapping to metal (e.g., titanium) density values.

(c) Template generation

At 540 in FIG. 5, based on base material density volume data 521 in the volume space, first computer system 370 may perform template extraction to generate template(s) 550 representing material property = base material thickness. Template generation may depend on target contour(s) and the selected material property = material thickness. For example, block 540 may involve masking projected material thickness 524 ($t_m^{\alpha}$) with projected target contour associated with target structure 133 along with a margin (e.g., for movement or shape change of target structure 133).

In one example, template 550 may be a soft-tissue template image (see 551) that is generated based on water density volume data (see 521) and associated projected water-equivalent thickness data (not shown). In another example, template 550 may be a bone-tissue template image (see 552) that is generated based on bone density volume data (see 523) and associated tissue-equivalent thickness (not shown). Resulting template 551/552 may also be referred to as projected template thickness. In practice, a set of $K$ templates may be generated for all gantry angles, such as $K$ = 360 templates for a full rotation of gantry 311 of imaging system 310. Each template 550 may be generated to be matchable against second material property data 580 that also represents the same material property for the purpose of target structure tracking during treatment phase 502.

(d) Template matching

At 590 in FIG. 5, during treatment phase 502, target structure tracking may be implemented by performing template matching based on template 550 and second material property data 580 representing the same material property. In one example, template 550 may be a soft-tissue template image (see 551) that is matchable against second material property data 580 representing water-equivalent thickness for soft tissue tracking (see 581). In another example, template 550 may be a bone-tissue template image (see 552) that is matchable against second material property data 580 representing bone-equivalent thickness for bony anatomy tracking (see 582).

Depending on the desired implementation, template matching 590 may involve calculating a normalized cross correlation within a specific search region around the isocenter, which results in a match score value between 0 and 1. Calculating the match score at different pixel offsets produces a match score surface defined over the search region. A possible match may be indicated by the highest peak in the match score surface. However, in practice, this may be an incorrect match (especially for noisy images with little contrast). For example, every match score surface would include a highest peak which does not necessarily mean there is a true match. For each match, a peak-to-sidelobe-ratio (PSR) may also be calculated, where the PSR is the peak value divided by the standard deviation of the sidelobes. A minimum threshold for the match score value and PSR may be used to reject likely false matches. The result of template matching 590 may include 2D position data associated with the relevant target structure (e.g., lung tumor, bony anatomy).

Image data processing (base material density/thickness)

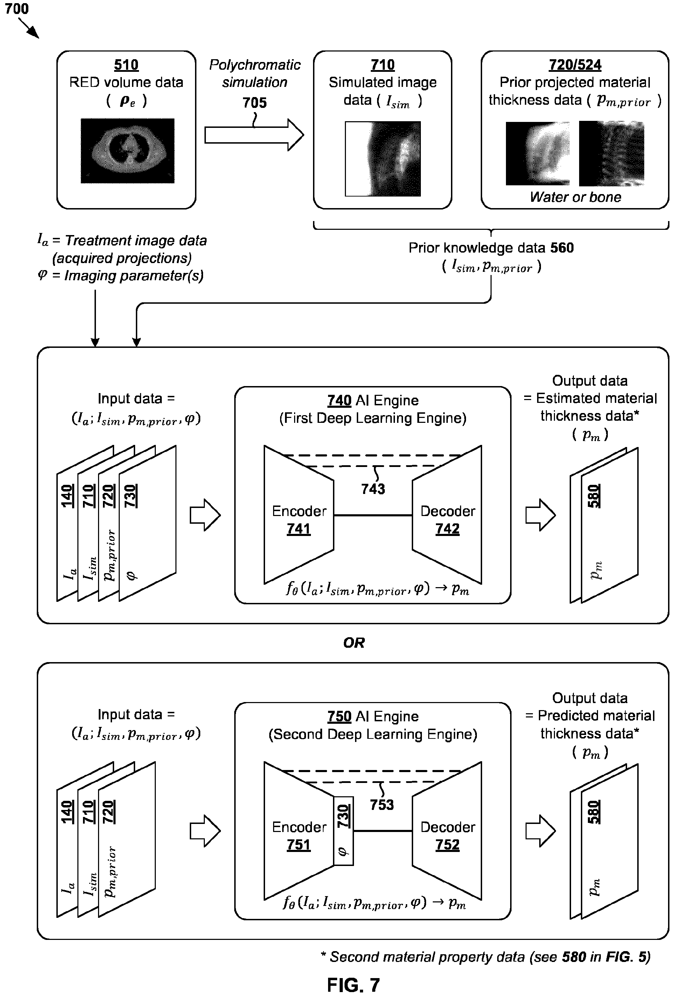

Blocks 560-580 in FIG. 5 will be explained further using FIG. 7, which is a flow diagram illustrating detailed example process 700 for image data processing using an AI engine to generate second material property data in the example in FIG. 5. Example process 700 may include one or more operations, functions, or actions illustrated by one or more blocks. The various blocks may be combined into fewer blocks, divided into additional blocks, and/or eliminated based upon the desired implementation. Second computer system 470 in FIG. 4 may be deployed to implement the example in FIG. 7.

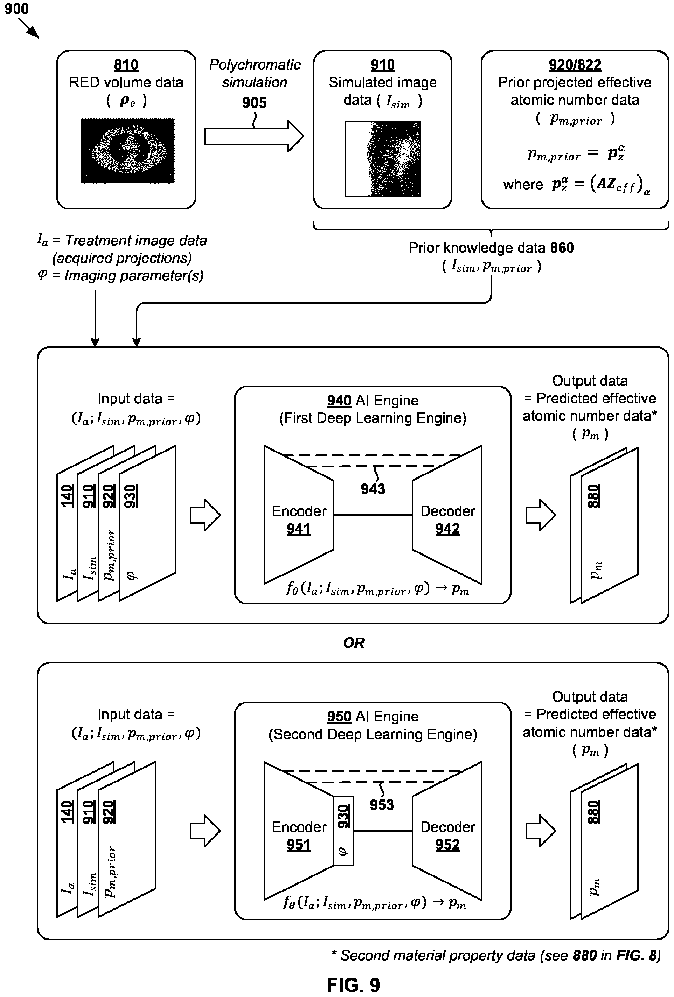

In practice, image data processing (see FIGs. 7 and 9) according to examples of the present disclosure may be implemented to enhance treatment image data 140 (e.g., 2D projection images), such as to increase the visibility/contrast of target structure 133 as represented using a particular material property. This may in turn improve the ability to detect target structure 133 in 2D projection images during treatment phase 502. By enhancing the quality of treatment image data 140 through the extraction of second material property data 580, examples of the present disclosure may be implemented to reduce the likelihood of tracking accuracy being negatively impacted by low contrast of target structure 133 (e.g., soft tissue occluded by bony anatomy).

As used herein, the term "AI engine" may refer to any suitable hardware and/or software component(s) of a computer system that are capable of executing algorithms according to any suitable AI model(s). Depending on the desired implementation, "AI engine" may be a machine learning engine based on machine learning model(s), deep learning engine based on deep learning model(s), etc. In general, deep learning is a subset of machine learning in which multi-layered neural networks may be used for feature extraction as well as pattern analysis and/or classification. The term "deep" in deep learning generally refers to the number of layers in the neural network. For example, compared to shallow learning models, deep learning models may have dozens or even hundreds of layers, for example dozens or hundreds of "hidden" layers between an input layer and an output layer. This allows deep learning models to extract more complex and nuanced features from the input image data, leading to better tracking accuracy and performance in radiation therapy.

Depending on the desired implementation, any suitable AI model(s) may be used to implement examples of the present disclosure, such as convolutional neural network (CNN), recurrent neural network (RNN), deep belief network, generative adversarial network (GAN), autoencoder(s), variational autoencoder(s), long short-term memory architecture for tracking purposes, or any combination thereof, etc. In practice, a neural network is generally formed using a network of processing elements (called "neurons," "nodes," etc.) that are interconnected via connections (called "synapses," "weight data," etc.). A processing layer of a convolutional neural network may be a convolutional layer, pooling layer, un-pooling layer, rectified linear units (ReLU) layer, fully connected layer, loss layer, activation layer, dropout layer, transpose convolutional layer, concatenation layer, or any combination thereof, etc. For example, CNNs may be implemented using any suitable architecture(s), such as UNet, LeNet, AlexNet, ResNet, VNet, DenseNet, OctNet, etc.

(a) Prior knowledge data

At 560 in FIG. 5 and 710-720 in FIG. 7, second computer system 470 may generate prior knowledge data 560 based on planning image data 110 and/or RED volume data 510 (denoted as $\rho_e$). In one example, prior knowledge data 560 may include (a) simulated projection image data 710 ($I_{sim}$) and (b) prior material thickness data 720 ($p_{m,prior}$) = projected material thickness images ($t_m^\alpha$) 524. In practice, $I_{sim}$ may be generated by performing polychromatic simulation (see 705 in FIG. 7) on RED volume data 510 ($\rho_e$). This way, additional information from polychromatic simulation 705 may be used to guide model prediction by AI engine 740/750 in FIG. 7 (to be discussed below).

Simulated projection image data ($I_{sim}$) 710 may be simulated intensity image $(I_k^\alpha)$ that is generated based on

RED volume data ($\rho_e$) 510 using any suitable polychromatic attenuation model, such as:

$$I_k^\alpha = \sum_{j=1}^{N_k} I_0^k(E_j) e^{-[A\mu(\rho_e, E_j)]_\alpha}.$$ (Eq. 1)

In equation (1), $\mu(\rho_e, E_j)$ denotes a polychromatic attenuation model for transforming $\rho_e$ into coefficients $\mu$; $I_0^k(E_j)$ denotes the energy resolved at norm; $\propto$ denotes a particular gantry angle; and $k$ denotes the low ($k = L$) or high ($k = H$) energy spectrum or is dropped in case of single energy acquisition.

(b) AI engine(s)

At 570 in FIG. 5, second computer system 470 may process input data = treatment image data 140 and prior knowledge data 560 to generate predicted output data = second material property data 580 using AI engine(s), such as deep learning engine 740/750 in FIG. 7. Here, deep learning engine 740/750 may be designed to be "physics informed" to map X-ray intensity data in treatment image data 140 to second material property data 580 representing base material thickness passed by radiation beam. This way, during template matching, second material property data 580 may be directly compared with template(s) 550 to improve tracking accuracy because both represent the same material property. This should be contrasted against conventional approaches that compare different physical quantities and/or rely on weighted log subtraction that is usually limited to dual energy images.

In the example in FIG. 7, deep learning engine 740/750 uses polychromatic forward simulation 705 of attenuation projection as well as base material thickness for a particular gantry angle given pre-treatment RED volume data 510. This way, deep learning engine 740/750 may leverage an explicit physics model as well as prior knowledge data 560 from planning phase 501 to predict the base material thickness for an incoming projection. Prior knowledge data 560 may be used as additional input channel(s) to serve as a constraint on the predicted base material thickness given the measured projection.

In general, the problem to be solved by deep learning engine 740/750 may be formulated as a mapping that is defined as follows:

$$f_\theta(I_a; I_{sim}, p_{m,prior}, \varphi) \to p_m, \text{ where } p_{m,prior} = t_m^\alpha.$$

Here, deep learning engine 740/750 may be trained to learn model ($f_\theta$) parameterized by $\theta$ (deep neural networks) to, given prior knowledge data 560 = ($I_{sim}, p_{m,prior}, \varphi$), map $I_a$ (i.e., treatment image data 140) to $p_m$ (i.e., second material property data 580). In practice, $I_a$ may include acquired SE or DE CBCT projection image data representing intensity or log-normalized attenuations. $I_{sim}$ denotes simulated projection image data (see 710) under the same or similar imaging parameters encoded by $\varphi$ (see 730), such as gantry angle $\alpha$, energy resolved air norm $I_0^k(E_j)$, etc. $p_{m,prior}$ denotes prior projected material property data, such as projected material thickness data ($t_m^\alpha$) 524 (e.g., bone/water thickness).

Deep learning engine 740/750 may be implemented using any suitable deep neural network architecture for deep regression, etc. In general, deep learning engine 740/750 may include a hierarchy of multiple ($N$) processing layers denoted as $L_1$ to $L_N$. Each layer is associated with a set of weights, collectively denoted as $w_{L1}$ to $w_{LN}$. One example architecture may include (a) encoder 741/751 to learn a representative latent space and (b) decoder 742/752 to recover expected output. Depending on the desired implementation, encoder 741/751 may be neural network layer(s) for extracting feature(s) from the input data. Decoder 742/752 may be neural network layer(s) for processing the extracted feature(s) from encoder 741/742 to generate the output data. Two examples are shown in FIG. 7.

(a) Input as channels: In one example, first deep learning engine 740 having multiple channels as input may be deployed. In this case, imaging parameters $\varphi$ may be expanded into a 2D array (e.g., by repetition) and stacked with ($I_a; I_{sim}, p_{m,prior}$) to form a multi-channel input = ($I_a; I_{sim}, p_{m,prior}, \varphi$) to encoder 741. Output data ($p_m$) from decoder 742 may be a multi-channel image representing the predicted material property associated with 2D projections ($I_a$) acquired during treatment phase 502.

(b) Channels plus latent vectors: Alternatively, second deep learning engine 750 having multiple channels plus latent vector(s) may be deployed. Here, instead of stacking the 2D array representing expanded $\varphi$ with image data ($I_a; I_{sim}, p_{m,prior}$), the 2D array may be stacked with latent vector(s) in a much lower dimension at the bottleneck of encoder 751. Note that a shallow set of fully connected layers may be applied to the original $\varphi$ to map it to the dimension of the encoder latent space. In practice, the "latent space" may be a representation of compressed data that is generated by encoder 751 and stored in latent vector(s).

Any suitable encoder architecture may be implemented, such as a CNN-based encoder used in UNet, transformer(s) in vision transformer (ViT) or any of their variants. The ViT model represents an input image as a series of image patches similar to a series of word embeddings for text-based processing. Any suitable decoder architecture may be used, provided the final output matches the expected dimensions. Dashed lines 743/753 in FIG. 7 may represent (optional) skip connections between encoder 741/751 and decoder 742/752. In general, skip connection 743/753 is a direct connection between encoder 741/751 and decoder 742/752 to allow decoder 742/752 to access data from encoder 741/751. Skip connection 743/753 is also known as a residual connection because it allows deep learning engine 740/750 to learn the residual mapping between the input data and output data.

Using examples of the present disclosure, deep learning engine 740/750 may be designed to be physics informed and trained to perform material decomposition in the projection space by learning model $f_\theta$, which is conditioned on prior knowledge data 560 = ($I_{sim}$, $p_{m,prior}$, $\varphi$). The output of deep learning engine 740/750 is second material property data 580 representing material property = base material thickness ( $t_m^\alpha$ ), such as bone thickness, water thickness, etc. Second material property data 580 may represent an enhanced form of treatment image data 140 where the visibility/contrast of target structure 133 is improved.

Template generation: effective atomic number

[0067]    A second example material property is effective atomic number ($Z_{eff}$). In the following, example template generation will be described using FIG. 8, which is a flow diagram illustrating a detailed example for target structure tracking based on material property in the form of effective atomic number. Example process 800 may include one or more operations, functions, or actions illustrated by one or more blocks. The various blocks may be combined into fewer blocks, divided into additional blocks, and/or eliminated based upon the desired implementation.

In practice, different tissue types generally have varying electron densities and interact differently with radiation. One example use case may be tracking target structures that are associated with high effective atomic number ($Z_{eff}$), such as tumor infused with iodine contrast, metal structure(s), etc.. In this example, during planning phase 801, template generation and first material property data generation may be performed according to blocks 810-850 (related to 210-250 in FIG. 2). During treatment phase 802, image processing to generate second material property data and template matching may be performed according to blocks 860-890 (related to 260-290 in FIG. 2). First computer system 370 in FIG. 3 may be deployed to implement blocks 810-850, and second computer system 470 in FIG. 4 to implement 860-890.

(a) Transformed image data and first material property data

At 810-830 in FIG. 8, first computer system 370 may obtain or generate RED volume data (denoted as $\rho_e$) and first material property data that includes (a) effective atomic number volume data (denoted as $Z_{eff}$) 821 and (b) projected effective atomic number data (denoted as $p_z^\alpha$ ) 822. Any suitable approach may be used to map the planning CT data to ($\rho_e$, $Z_{eff}$). Similar to the example in FIG. 5, RED volume data 810 may be a 3D representation of the electron density distribution within a patient's anatomy, expressed as a normalized value relative to water. Various explanations relating to RED volume data 510 in FIG. 5 are also applicable here and will not be repeated for brevity.

Projected effective atomic number data ( $p_z^\alpha$ ) 822 (i.e., 2D) may be generated by performing a forward projection based on effective atomic number volume data 821 (i.e., 3D). This may involve forward projecting RED volume data ($\rho_e$) 810 and effective atomic number volume data ($Z_{eff}$) 830 using a forward-projection operator ($A$) to obtain a line integral of the $\rho_e$, and $Z_{eff}$ as seen by a ray passing through the volume towards a particular pixel at the detector for a particular gantry angle ($\alpha$):

$$p_z^\alpha = \left( A Z_{eff} \right)_\alpha. \qquad \text{(Eq. 2)}$$

According to a publication entitled "Energy-selective reconstructions in X-ray computerized tomography" by Alvarez R. E. and Macovski A. (see Phys Med Biol. 1976 Sep; 21(5):733-54) and incorporated herein by reference, energy-dependent attenuation $\mu(E)$ may be approximated using the equation below:

$$\mu(E) = c_1 \rho_e Z_{eff}^n E^{-3} + c_2 \rho_e f_{KN}(E). \qquad \text{(Eq. 3)}$$

In equation (3), $\rho_e$ denotes the RED value, $f_{KN}(E)$ denotes the Klein-Nishina function specific to a material, $c_1$, $c_2$, and $n$ are model parameters obtained by fitting the model to data. By knowing the spectral properties of the CBCT imaging system, equation (3) may be used to map the $\rho_e$ and $Z_{eff}$ volume data obtained from planning image data 110 (e.g., planning CT) into measured intensities on treatment image data 140 (e.g., CBCT projections) for the low and high energy acquisition $I_L$ and $I_H$, respectively:

$$I_k^\alpha = \sum_{j=1}^{N_k} I_0^k(E_j) e^{-[A\mu(E_j)]_\alpha}. \qquad \text{(Eq. 4)}$$

In equation (4), $k$ denotes the low energy index (i.e., $k = L$ in $I_L$) or high energy index (i.e., $k = H$ in $I_H$). Further, $I_0^k(E_j)$ denotes the energy resolved air norm, $A$ is the forward-projection operator, $\alpha$ is the gantry angle and $\mu(E)$ is calculated from the approximation in equation (3).

(b) Template generation

At 840 in FIG. 8, first computer system 370 may perform template extraction to generate template 550 representing material property = effective atomic number associated with target structure 133 to be tracked. For example, at 841, to increase the visibility of targets with high atomic number, projected background effective atomic number data may be generated by replacing original target structure $Z_{eff}$ values by water within the target structure volume:

$$p_{z,b}^\alpha = \left(A Z_{eff}^b\right)_\alpha. \qquad \text{(Eq. 5)}$$

At 842 in FIG. 8, first computer system 370 may generate a template representing material property = effective atomic number for enhanced target structure based on projected effective atomic number data ( $p_z^\alpha$ ) and projected background effective atomic number data ( $p_{z,b}^\alpha$ ) as follows:

$$p_{z,template}^\alpha = p_z^\alpha - p_{z,b}^\alpha. \qquad \text{(Eq. 6)}$$

(c) Template matching

At 890 in FIG. 8, during treatment phase 802, target structure tracking may be implemented by performing template matching based on template 850 ( $p_{z,template}^\alpha$ ) and second material property data 880 ($p_m$) representing the same material property = effective atomic number. Similar to the example in FIG. 5, template matching 890 may involve calculating a normalized cross correlation, etc. Various explanations relating to block 590 in FIG. 5 are also applicable here and will not be repeated for brevity.

Image data processing (effective atomic number)

Blocks 860-880 in FIG. 8 will be explained further using FIG. 9, which is a flow diagram illustrating detailed example 900 for image data processing using an AI engine to generate second material property data in the example in FIG. 8. Example process 900 may include one or more operations, functions, or actions illustrated by one or more blocks. The various blocks may be combined into fewer blocks, divided into additional blocks, and/or eliminated based upon the desired implementation. Second computer system 470 in FIG. 4 may be deployed to implement the example in FIG. 9.

(a) Prior knowledge data

At 860 in FIG. 8 and 910-920 in FIG. 9, second computer system 470 may generate prior knowledge data 860 based on planning image data 110 and/or RED volume data 810 ($\rho_e$). In one example, prior knowledge data 560 may include (a) simulated projection image data 810 ($I_{sim}$) and (b) prior projected effective atomic number data 820 ($p_{m,prior}$) in the form of projected effective atomic number data 822 ( $p_z^\alpha$ ) in equation (2). Similar to the example in FIG. 7, $I_{sim}$ may be generated by performing polychromatic simulation 905 based on RED volume data 810 ($\rho_e$) and effective atomic volume data ($Z_{eff}$) 821 from planning CT 110, such as using the example polychromatic attenuation model in equation (4). $I_{sim}$ may also be referred to as simulated intensity image data.

(b) AI engine(s)

At 870 in FIG. 8, second computer system 470 may process input data = treatment image data 140 and prior knowledge data 860 to generate predicted output data = second material property data 880 using AI engine(s), such as deep learning engine 940/950 in FIG. 9. Here, deep learning engine 940/950 may be designed to be "physics informed" to map X-ray intensity data in treatment image data 140 to second material property data 880 representing effective atomic number. During template matching, second material property data 880 may be directly compared with template(s) 850 to improve tracking accuracy because both represent the same material property.

Similar to the example in FIG. 7, deep learning engine 940/950 may be trained to learn model ($f_\theta$) below that is parameterized by $\theta$ (deep neural networks) to map $I_a$ to $p_m$ given prior knowledge data 860. Here, $I_a$ denotes acquired SE or DE CBCT projection image data. $I_{sim}$ denotes simulated projection image data (see 910) under the same or similar imaging parameters encoded by $\varphi$ (see 930), such as gantry angle $\alpha$, energy resolved air norm $I_0^k(E_j)$, etc. $p_{m,prior}$ denotes prior material property data 920 = projected effective atomic number data ( $p_z^\alpha$ ) in equation (4):

$$f_\theta(I_a; I_{sim}, p_{m,prior}, \varphi) \rightarrow p_m, \text{ where } p_{m,prior} = p_z^\alpha.$$

Similar to the example in FIG. 7, deep learning engine 940/950 may include (a) encoder 941/951 to learn a representative latent space and (b) decoder 942/952 to recover expected output. Any suitable encoder/decoder architecture may be implemented. Also, skip connections 943/953 connecting encoder 941/951 and decoder 942/952 are optional. Two example implementations are shown in FIG. 9.

(a) Input as channels: In one example, first deep learning engine 940 having multiple channels as input may be deployed. In this case, imaging parameters $\varphi$ may be expanded into a 2D array (e.g., by repetition) and stacked with ($I_a$; $I_{sim}$, $p_{m,prior}$) to form a multi-channel input = ($I_a$; $I_{sim}$, $p_{m,prior}$, $\varphi$) to encoder 941. Output data ($p_m$) from decoder 942 may be a multi-channel image representing projected effective atomic number data 880 associated with $I_a$ acquired during treatment phase 802.

(b) Channels plus latent vectors: Alternatively, second deep learning engine 950 having multiple channels plus latent vector(s) may be deployed. Compared to first deep learning engine 940, the 2D array representing expanded $\varphi$ may be stacked with latent vector(s) in a much lower dimension at the bottleneck of encoder 951.

The output of deep learning engine 940/950 is second material property data 880 ($p_m$) representing material property = effective atomic number. Second material property data 880 may represent an enhanced form of treatment image data 140 where the visibility/contrast of target structure 133 is improved to improve tracking accuracy and treatment outcomes for patient 320. During template matching at block 890 in FIG. 8, second material property data 880 may be directly compared with template 850 because both represent the same material property = effective atomic number. Again, this should be contrasted against conventional approaches that compare different physical quantities/properties.

For acquired low and high energy projections $I_L$ and $I_H$, second material property data 880 denoted as $p_z^\alpha(\mu_L, \mu_H)$ may be estimated using model ($f_\theta$) learned by deep learning engine 940/950. Note that the transformation from measured intensities $I$ to the attenuation $\mu$ is given by log-normalization step: $\mu = -log\frac{I}{I_0}$ and the corresponding air norm $I_0$ is generally known for a particular acquisition. By subtracting the projected background effective atomic number $p_z^\alpha(\mu_L, \mu_H)$-$p_{z,b}^\alpha$ , target-enhanced image data may be generated and subsequently compared with the template $p_{z,template}^\alpha$ in the template-based matching procedure at block 890.

AI engine training

Deep learning engine 740/750/940/950 may be trained prior to treatment delivery phase 502/802 using any suitable approach, such as supervised learning, unsupervised learning, semi-supervised learning, reinforcement learning, a combination thereof, etc. Using supervised learning, deep learning engine 740/750/940/950 may be trained using labeled training data that includes labeled examples in order to learn model ($f_\theta$) that maps the input data = ($I_a$; $I_{sim}$, $p_{m,prior}$, $\varphi$) to the desired output data = $p_m$. In contrast, using unsupervised learning, deep learning engine 740/750/940/950 may be trained using unlabeled training data to uncover hidden patterns and structures within the data.

Using semi-supervised learning, a combination of labelled training data and (possibly a larger set of) unlabeled training data may be used to train deep learning engine 740/750/940/950. In this case, deep learning engine 740/750/940/950 may use the labeled training data to learn a basic understanding of model ($f_\theta$) and then leverage the unlabeled training data to refine model ($f_\theta$). For the semi-supervised approach, the measured intensities in the acquired projections may be compared with estimated intensities from the projected material thickness data 580 in FIG. 7 (or projected RED and projected effective atomic number data 880 in FIG. 9). For example, in the case of inferred water/bone thickness, the intensity estimate may be calculated as follows:

$$\widehat{I_k^\alpha} = \sum_{j=1}^{N_k} I_0^k(E_j) exp\left[-\sum_m \widehat{t_m^\alpha}\mu_m(E_j)\right]. \qquad \text{(Eq. 7)}$$

In equation (7), m is material index (bone/water), $\widehat{t_m^\alpha}$ is the estimated material thickness in the CBCT projection, $\mu_m(E_j)$ denotes the material specific attenuation curve, $k$ denotes the low/high energy spectrum or is dropped in case of single energy acquisition. Possible loss function could be a mean squared error (MSE) between measured $I_k^\alpha$ (i.e., treatment image data 140) and the estimated $\widehat{I_k^\alpha}$.

*Example*

The present disclosure provides a method for generating material property data associated with an image comprising an anatomical structure to be tracked. The material property data is of a particular material property. Image data may be processed using an artificial intelligence engine to generate the material property data for use with a template that also represents the same particular material property. Template matching using the template and the material property data of the image may be performed to track motion of the anatomical structure. In some examples, the material property may be material density, material thickness, or effective atomic number.

This way, during template matching, material property data of the image may be directly compared with the template to improve tracking accuracy because both represent the same material property.

This may be contrasted against conventional approaches that compare different physical quantities and/or rely on weighted log subtraction that is usually limited to dual energy images.

Computer system

The above examples can be implemented by hardware (including hardware logic circuitry), software or firmware or a combination thereof. The above examples may be implemented by any suitable computing device, computer system, etc. The computer system may include processor(s), memory unit(s) and physical NIC(s) that may communicate with each other via a communication bus, etc. The computer system may include a non-transitory computer-readable medium having stored thereon instructions or program code that, when executed by the processor, cause the processor to perform processes described herein with reference to the drawings.

The techniques introduced above can be implemented in special-purpose hardwired circuitry, in software and/or firmware in conjunction with programmable circuitry, or in a combination thereof. Special-purpose hardwired circuitry may be in the form of, for example, one or more application-specific integrated circuits (ASICs), programmable logic devices (PLDs), field-programmable gate arrays (FPGAs), and others. The term 'processor' is to be interpreted broadly to include a processing unit, ASIC, logic unit, or programmable gate array etc.

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or any combination thereof.

Those skilled in the art will recognize that some aspects of the embodiments disclosed herein, in whole or in part, can be equivalently implemented in integrated circuits, as one or more computer programs running on one or more computers (e.g., as one or more programs running on one or more computing systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure.

Software to implement the techniques introduced here may be stored on a non-transitory computer-readable storage medium and may be executed by one or more general-purpose or special-purpose programmable microprocessors. A "computer-readable storage medium", as the term is used herein, includes any mechanism that provides (i.e.,

stores and/or transmits) information in a form accessible by a machine (e.g., a computer, network device, personal digital assistant (PDA), mobile device, manufacturing tool, any device with a set of one or more processors, etc.). A computer-readable storage medium may include recordable/non recordable media (e.g., read-only memory (ROM), random access memory (RAM), magnetic disk or optical storage media, flash memory devices, etc.).

The drawings are only illustrations of an example, wherein the units or procedure shown in the drawings are not necessarily essential for implementing the present disclosure. Those skilled in the art will understand that the units in the device in the examples can be arranged in the device in the examples as described or can be alternatively located in one or more devices different from that in the examples. The units in the examples described can be combined into one module or further divided into a plurality of sub-units.

CLAUSES

The following numbered clauses are subject-matter of, but not currently claims of, the present EP application. **That is, the following clauses are part of the description, and are not claims.** The Applicant reserves the right to claim this subject-matter in this EP application.

1. A method for a computer system to perform image data processing for target structure tracking, wherein the method comprises:

obtaining treatment image data associated with a target structure of a patient requiring radiation therapy, wherein the treatment image data is acquired using an imaging system during a treatment phase of the radiation therapy; and

processing the treatment image data using an artificial intelligence (AI) engine to generate material property data representing a particular material property associated with the target structure, wherein the material property data is matchable against a template that also represents the particular material property for tracking the target structure based on the particular material property during the treatment phase.

2. The method of clause 1, wherein processing the treatment image data comprises:
processing the treatment image data using the AI engine to generate the material property data representing the particular material property in the form of material density or material thickness.

3. The method of clause 1, wherein processing the treatment image data comprises:
processing at least the treatment image data using the AI engine to generate the material property data representing the particular material property in the form of effective atomic number associated with the target structure.

4. The method of clause 1, wherein processing the treatment image data comprises:

obtaining prior knowledge data that is generated based on planning image data associated with the target structure, wherein the planning image data is acquired prior to the treatment phase of the radiation therapy; and

processing the treatment image data and the prior knowledge data using the AI engine to generate the material property data representing the particular material property.

5. The method of clause 4, wherein the method further comprises:
generating the prior knowledge data that includes simulated projection image data by performing polychromatic simulation based on transformed image data that is generated based on the planning image data.

6. The method of clause 4, wherein the method further comprises:
generating the prior knowledge data that includes prior material property data representing the particular material property based on the planning image data or transformed image data that is generated based on the planning image data.

7. The method of clause 4, wherein processing the treatment image data comprises:
processing the treatment image data and the prior knowledge data using the AI engine that includes an encoder and a decoder to generate the material property data representing the particular material property.

8. A method for a computer system to perform target structure tracking for radiation therapy, wherein the method comprises:

obtaining material property data that represents a particular material property associated with a target structure of a patient requiring radiation therapy, wherein the material property data is generated using an artificial intelligence (AI) engine based on treatment image data acquired during a treatment phase of the radiation therapy; and obtaining a template that also represents the particular material property associated with the target structure, wherein the template is generated based on (a) planning image data that is acquired prior to the treatment phase or (b) transformed image data that is generated based on the planning image data; and based on the material property data and the template, performing template matching during the treatment phase to track the target structure based on the particular material property.

9. The method of clause 1 or clause 8, wherein performing the template matching comprises:
performing the template matching based on the material property data and the template that both represent the particular material property in the form of material thickness associated with the target structure.

10. The method of clause 1 or clause 8, wherein performing the template matching comprises:
performing the template matching based on the material property data and the template that both represent the particular material property in the form of effective atomic number associated with the target structure.

11. The method of clause 1 or clause 8, wherein the method further comprises:
processing the treatment image data using the AI engine to generate the material property data.

12. The method of clause 11, wherein processing the treatment image data comprises:

obtaining prior knowledge data that is generated based on the planning image data associated with the target structure; and
processing the treatment image data and the prior knowledge data using the AI engine to generate the material property data representing the particular material property.

13. The method of clause 12, wherein processing the treatment image data comprises:
generating the prior knowledge data that includes simulated projection image data by performing polychromatic simulation based on the transformed image data.

14. The method of clause 12, wherein processing the treatment image data comprises:
generating the prior knowledge data that includes prior material property data representing the particular material property based on the planning image data or the transformed image data.

15. A computer system, comprising:

a processor; and
a non-transitory computer-readable medium having stored thereon instructions that, when executed by the processor, cause the processor to perform the following:

obtain treatment image data associated with a target structure of a patient requiring radiation therapy, wherein the treatment image data is acquired using an imaging system during a treatment phase of the radiation therapy; and
process the treatment image data using an artificial intelligence (AI) engine to generate material property data representing a particular material property associated with the target structure, wherein the material property data is matchable against a template that also represents the particular material property for tracking the target structure based on the particular material property during the treatment phase.

16. The computer system of clause 15, wherein the instructions for processing the treatment image data cause the processor to:
process the treatment image data using the AI engine to generate the material property data representing the particular material property in the form of material density or material thickness.

17. The computer system of clause 15, wherein the instructions for processing the treatment image data cause the processor to:
process at least the treatment image data using the AI engine to generate the material property data representing the particular material property in the form of effective atomic number associated with the target structure.

18. The computer system of clause 15, wherein the instructions for processing the treatment image data cause the processor to:

obtain prior knowledge data that is generated based on planning image data associated with the target structure, wherein the planning image data is acquired prior to the treatment phase of the radiation therapy; and process the treatment image data and the prior knowledge data using the AI engine to generate the material property data representing the particular material property.

19. The computer system of clause 18, wherein the instructions further cause the processor to: generate the prior knowledge data that includes simulated projection image data by performing polychromatic simulation based on transformed image data that is generated based on the planning image data.

20. The computer system of clause 18, wherein the instructions further cause the processor to: generate the prior knowledge data that includes prior material property data representing the particular material property based on the planning image data or transformed image data that is generated based on the planning image data.

21. The computer system of clause 18, wherein the instructions for processing the treatment image data cause the processor to: process the treatment image data and the prior knowledge data using the AI engine that includes an encoder and a decoder to generate the material property data representing the particular material property.

**Claims**

1. A method for a computer system to perform image data processing for tracking of an anatomical structure of a subject, wherein the method comprises:

obtaining image data associated with the anatomical structure of the subject, wherein the image data is acquired using an imaging system; processing the image data using an artificial intelligence (AI) engine to generate material property data representing a particular material property associated with the anatomical structure, wherein the material property data is matchable against a template that also represents the particular material property for tracking the anatomical structure based on the particular material property.

2. The method of claim 1, wherein processing the image data comprises: processing the image data using the AI engine to generate the material property data representing the particular material property in the form of material density or material thickness.

3. The method of claim 1 or claim 2, wherein processing the image data comprises: processing at least the image data using the AI engine to generate the material property data representing the particular material property in the form of effective atomic number associated with the anatomical structure.

4. The method of any preceding claim, wherein the anatomical structure comprises a target structure of a patient requiring radiation therapy, and the processing of the image data comprises:

obtaining prior knowledge data that is generated based on planning image data associated with the anatomical structure, wherein the planning image data is acquired prior to a treatment phase of the radiation therapy; and processing the image data and the prior knowledge data using the AI engine to generate the material property data representing the particular material property.

5. The method of claim 4, wherein the method further comprises: generating the prior knowledge data that includes simulated projection image data by performing polychromatic simulation based on transformed image data that is generated based on the planning image data.

6. The method of claim 4, wherein the method further comprises: generating the prior knowledge data that includes prior material property data representing the particular material property based on the planning image data or transformed image data that is generated based on the planning image data.

7. The method of claim 4, wherein processing the image data comprises:
processing the image data and the prior knowledge data using the AI engine that includes an encoder and a decoder to generate the material property data representing the particular material property.

8. The method of any preceding claim, wherein the method further comprises obtaining the template that also represents the particular material property associated with the anatomical structure, wherein the template is generated based on (a) planning image data that is acquired prior to a treatment phase of radiotherapy of the subject or (b) transformed image data that is generated based on the planning image data; and
based on the material property data and the template, performing template matching to track the anatomical structure based on the particular material property.

9. The method of claim 8, wherein performing the template matching comprises:
performing the template matching based on the material property data and the template that both represent the particular material property in the form of material density or material thickness associated with the anatomical structure.

10. The method of claim 8 or claim 9, wherein performing the template matching comprises:
performing the template matching based on the material property data and the template that both represent the particular material property in the form of effective atomic number associated with the anatomical structure.

11. A computer system, comprising:

a processor; and
a non-transitory computer-readable medium having stored thereon instructions that, when executed by the processor, cause the processor to perform the following:

obtain image data associated with an anatomical structure of a subject, wherein the image data is acquired using an imaging system;
process the image data using an artificial intelligence (AI) engine to generate material property data representing a particular material property associated with the anatomical structure, wherein the material property data is matchable against a template that also represents the particular material property for tracking the anatomical structure based on the particular material property.

12. The computer system of claim 11, wherein the instructions for processing the image data cause the processor to:
process the image data using the AI engine to generate the material property data representing the particular material property in the form of material density or material thickness.

13. The computer system of claim 11 or claim 12, wherein the instructions for processing the image data cause the processor to:
process at least the image data using the AI engine to generate the material property data representing the particular material property in the form of effective atomic number associated with the anatomical structure.

14. The computer system of any of claim 11 to claim 13, wherein the instructions cause the processor to perform the method of any of claims 4 to 10.

15. The computer system of any of claim 11 to claim 14, further configured to:

acquire the image data during a treatment phase of radiation therapy in which radiation therapy treatment is applied to the subject, wherein the image data comprises treatment image data; and
track the anatomical structure comprising a radiation therapy treatment target structure, during the radiation therapy treatment phase.

**100**

**101 PLANNING PHASE** | **102 TREATMENT PHASE**

**FIG. 1**

**200**

## 201 PLANNING PHASE

**210**
Obtain planning image data acquired during planning phase and/or transformed image data

> **110**
> Planning image data
> (e.g., planning CT)

> **211**
> Transformed image data
> (e.g., RED volume data)

**220**
Generate first material property data representing particular material property

> **221**
> Material property = base material density/thickness

> **222**
> Material property = effective atomic number

**230**
First material property data representing particular material property

**240**
Generate template(s) representing particular material property based on first material property data

**250**
Template(s) representing particular material property

> 359°
> 0°

## 202 TREATMENT PHASE

**260**
Obtain treatment image data acquired during treatment phase

> **140**
> Treatment image data (e.g., SE/DE CBCT projections)

> **261**
> Prior knowledge data

**270**
Generate second material property data representing particular material property

> **271**
> Material property = base material density/thickness

> **272**
> Material property = effective atomic number

**280**
Second material property data representing particular material property

**290**
Perform template matching based on template and second material property data representing the same particular material property

**FIG. 2**

**300**

Imaging system
**310**

Gantry
**311**

**312**

Radiation
source
**330**

Patient
**320**

313

Imaging
Beam
**350**

Detector
**340**

Control
Signal(s)
**361**

Planning Image Data
(e.g., planning CT)
**110**

Control System
**360**

Interface
**371**

Material Property Data
Generator **372**

Template Generator
**373**

Computer System **370**
(Template Generation)

**FIG. 3**

**FIG. 4**

**500**

**501 PLANNING PHASE**

**502 TREATMENT PHASE**

**110**
Planning image data
(e.g., planning CT)

**140**
Treatment image data
(e.g., SE or DE CBCT projections)

$I_a =$

**510**
Transformed image data =
physical property volume data

*E.g., RED
volume data*

$( \rho_e )$

**560**
Prior knowledge data

$I_{sim}$ = simulated
projection data

$p_{m,prior}$ = prior material
property data
(material
thickness $t_m^\alpha$ )

**520** Generate first material
property data

**521** Perform material
decomposition (volume space)
to generate material density
volume data ( $\rho_m$ )

522

523

*Water density or bone density*

**524*** $t_m^\alpha = (A\rho_m)_\alpha$

*\*A: Forward projection operator*

**570**
Perform material decomposition
(projection space) to generate
second material property data
using AI Engine

$L_1$   $L_2$   ...   $L_N$

$w_{L1}$   $w_{L2}$   ...   $w_{LN}$

$f_\theta(I_a; I_{sim}, p_{m,prior}, \varphi) \rightarrow p_m$

**530**
First material property data =
$( \rho_m , t_m^\alpha )$

**580**
Second material property data =
base material thickness data ( $p_m$ )

581

582

*Water or bone*

**540**
Perform template extraction to
generate template(s) representing
base material thickness

**550**
Template(s) representing base
material thickness

551
*Soft
tissue*

552
*Bone*

*Selected
template*

**590**
Perform template matching based
on template and second material
property data

**FIG. 5**

600

**FIG. 6**

**700**

**510**
RED volume data
( $\rho_e$ )

*Polychromatic simulation*
**705**

**710**
Simulated image
data ( $I_{sim}$ )

**720/524**
Prior projected material
thickness data ( $p_{m,prior}$ )

*Water or bone*

$I_a$ = Treatment image data
(acquired projections)
$\varphi$ = Imaging parameter(s)

Prior knowledge data **560**
( $I_{sim}, p_{m,prior}$ )

Input data =
( $I_a; I_{sim}, p_{m,prior}, \varphi$ )

140  710  720  730

$I_a$  $I_{sim}$  $p_{m,prior}$  $\varphi$

**740** AI Engine
(First Deep Learning Engine)

743

Encoder
**741**

Decoder
**742**

$f_\theta (I_a; I_{sim}, p_{m,prior}, \varphi) \to p_m$

Output data
= Estimated material
thickness data*
( $p_m$ )

580

$p_m$

**OR**

Input data =
( $I_a; I_{sim}, p_{m,prior}, \varphi$ )

140  710  720

$I_a$  $I_{sim}$  $p_{m,prior}$

**750** AI Engine
(Second Deep Learning Engine)

730

753

Encoder
**751**

$\varphi$

Decoder
**752**

$f_\theta (I_a; I_{sim}, p_{m,prior}, \varphi) \to p_m$

Output data
= Predicted material
thickness data*
( $p_m$ )

580

$p_m$

* Second material property data (see **580** in FIG. 5)

**FIG. 7**

**800**

**801 PLANNING PHASE** | **802 TREATMENT PHASE**

**110**
Planning image data
(e.g., planning CT)

**140**
Treatment image data
(e.g., SE or DE CBCT projections)

$I_a =$

**810**
Physical property volume data
= RED volume data ( $\rho_e$ )

**860**
Prior knowledge data

$I_{sim}$ = simulated
projection data

$p_{m,prior}$ = prior material
property data
( $p_z^\alpha$ )

**820**
Generate first material
property data

**821** Effective atomic number
volume data ( $Z_{eff}^n$ )

**822*** $p_z^\alpha = \left( A Z_{eff} \right)_\alpha$

**A:* Forward projection operator*

**830**
First material property data
( $Z_{eff}^n$ , $p_z^\alpha$ )

**840**
Perform template extraction to
generate template(s) representing
effective atomic number

**841** $p_{z,b}^\alpha = \left( A Z_{eff}^b \right)_\alpha$

**842** $p_{z,template}^\alpha = p_z^\alpha - p_{z,b}^\alpha$

**850**
Template(s) representing effective
atomic number ( $p_{z,template}^\alpha$ )

**870**
Process treatment image data and
prior knowledge data to generate
second material property data
using AI Engine

| L₁ | L₂ | ... | L_N |
| w_{L1} | w_{L2} | ... | w_{LN} |

$f_\theta (I_a; I_{sim}, p_{m,prior}, \varphi) \rightarrow p_m$

**880**
Second material property data =
effective atomic number data
( $p_m$ )

**890**
Perform template matching based
on template and second material
property data

**FIG. 8**

**900**

| **810**<br>RED volume data<br>( $\rho_e$ ) | *Polychromatic simulation* **905** | **910**<br>Simulated image data ( $I_{sim}$ ) | **920/822**<br>Prior projected effective atomic number data<br>( $p_{m,prior}$ )<br><br>$p_{m,prior} = p_z^\alpha$<br><br>where $p_z^\alpha = (AZ_{eff})_\alpha$ |

$I_a$ = *Treatment image data*
*(acquired projections)*
$\varphi$ = *Imaging parameter(s)*

Prior knowledge data **860**
( $I_{sim}, p_{m,prior}$ )

Input data =
$(I_a; I_{sim}, p_{m,prior}, \varphi)$

**940** AI Engine
(First Deep Learning Engine)

943

Encoder **941**     Decoder **942**

$f_\theta(I_a; I_{sim}, p_{m,prior}, \varphi) \rightarrow p_m$

Output data
= Predicted effective atomic number data*
( $p_m$ )

**880**

*OR*

Input data =
$(I_a; I_{sim}, p_{m,prior}, \varphi)$

**950** AI Engine
(Second Deep Learning Engine)

953

Encoder **951**   **930**   Decoder **952**

$f_\theta(I_a; I_{sim}, p_{m,prior}, \varphi) \rightarrow p_m$

Output data
= Predicted effective atomic number data*
( $p_m$ )

**880**

*\* Second material property data (see **880** in FIG. 8)*

**FIG. 9**

## EUROPEAN SEARCH REPORT

**Application Number**

EP 25 15 8537

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/061908 A1 (PAYSAN PASCAL [CH] ET AL) 22 February 2024 (2024-02-22) | 1,4-6,8, 11,14,15 | INV. G06T7/20 |
| Y | * abstract * <br> * figure 2 * <br> * paragraphs [0003] - [0005], [0007], [0009], [0044], [0052], [0055], [0057], [0065], [0085] * | 2,3,7,9, 10,12,13 | |
| X | YABO FU ET AL: "Enhancing the target visibility with synthetic target specific digitally reconstructed radiograph for intrafraction motion monitoring: A proof-of-concept study", MEDICAL PHYSICS, AIP, MELVILLE, NY, US, vol. 50, no. 12, 3 July 2023 (2023-07-03), pages 7791-7805, XP072545198, ISSN: 0094-2405, DOI: 10.1002/MP.16580 | 1,11 | |
| Y | * abstract * <br> * figures 1,2,12 * <br> * Sect. 1, 2.1, 2.2, 4 * <br> * page 7802, right-hand column, paragraph 2 * <br> * page 7792, right-hand column * | 2,3,12, 13 | |
| Y | US 2018/035965 A1 (SCHMIDT TAL GILAT [US] ET AL) 8 February 2018 (2018-02-08) * abstract * * paragraph [0019] * | 2,3,9, 10,12,13 | |
| Y | CN 114 403 912 A (SHENGPEAK MEDICAL SYSTEM SHARE LTD COMPANY) 29 April 2022 (2022-04-29) * abstract * * figure 3 * * 3rd paragraph of the summary section * | 2,3,7,9, 10,12,13 | |

**TECHNICAL FIELDS SEARCHED (IPC)**

G06T

The present search report has been drawn up for all claims

| Place of search | Date of completion of the search | Examiner |
|---|---|---|
| Munich | 16 July 2025 | Scholz, Volker |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                              

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 25 15 8537

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2025

| Patent document cited in search report | | Publication date | Patent family member(s) | | Publication date |
|---|---|---|---|---|---|
| US 2024061908 | A1 | 22-02-2024 | CN | 117083634 A | 17-11-2023 |
| | | | EP | 4315246 A1 | 07-02-2024 |
| | | | US | 2022309294 A1 | 29-09-2022 |
| | | | US | 2024061908 A1 | 22-02-2024 |
| | | | WO | 2022200283 A1 | 29-09-2022 |
| US 2018035965 | A1 | 08-02-2018 | US | 2015371378 A1 | 24-12-2015 |
| | | | US | 2018035965 A1 | 08-02-2018 |
| CN 114403912 | A | 29-04-2022 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 18586532 B

**Non-patent literature cited in the description**

- *Phys Med Biol.*, September 1976, vol. 21 (5), 733-54